# EUROPEAN PATENT SPECIFICATION

(11) **EP 0 479 376 B1**
(45) Date of publication and mention of the grant of the patent: **25.01.1995**
(21) Application number: 91202498.1
(22) Date of filing: 26.09.1991
(51) Int. Cl.: C07K 7/08, G01N 33/576, A61K 39/29

(54) **Peptides immunochemically reactive with antibodies directed against hepatitis Non-A, Non-B virus**
Mit Antikörper gegen Hepatitis-Virus non-A, non-B immunochemisch reagierende Peptide
Peptides agissants immunochimiquemeur avec des anticorps contre le virus d'hépatite non-A, non-B

(30) Priority: 05.10.1990 EP 90202658
(43) Date of publication of application: 08.04.1992
(73) Proprietor: Akzo Nobel N.V., 6824 BM Arnhem (NL)
(72) Inventor: Habets, Winand Johannes Antonius, NL-5232 JB 's-Hertogenbosch (NL)
(74) Representative: Hermans, Franciscus G.M.

(56) References cited:
- EP-A- 0 363 025
- EP-A- 0 388 232
- NUCLEIC ACIDS RESEARCH, vol. 18, no. 15, 11th August 1990, page 4626, Oxford University Press; K. TAKEUCHI et al.: "Nucleotide sequence of core and envelope genes of the hepatitis C virus genome derived directly from human healthy carriers"
- GASTROENTEROLOGY, vol. 25, no. 2, April 1990, pages 218-222, The Japanese Society of Gastroenterology, JP; T. ARIMA et al.: "A cDNA clone encoding a peptide highly specific for hepatitis C infection"

## Description

The invention relates to peptides which react immunochemically with antibodies directed against hepatitis Non-A, Non-B virus (NANBH-virus).

The invention further relates to a method for the detection of NANBH or anti-NANBH in a test fluid, and to an immunochemical reagent and a test kit which may be used in said detection method.

Non-A, Non-B hepatitis (which may or may not be caused by Hepatitis C Virus) is a transmittable disease or family of diseases shown to be virus-induced. It can be distinguished from other forms of viral-associated liver diseases, including that caused by the known hepatitis viruses, i.e., hepatitis A virus (HAV), hepatitis B virus (HBV), delta hepatitis virus (HDV), and hepatitis induced by cytomegalovirus (CMV) or Epstein-Barr virus (EBV). NANBH was first identified in transfused individuals. Transmission from man to chimpanzee and serial passage in chimpanzees provided evidence that NANBH is due to a transmissible infectious agent or agents. However, the transmissible agent responsible for NANBH is still not completely identified and the number of agents which are causative of the disease(s) are unknown.

Epidemiologic evidence is suggestive that there may be three types of NANBH: the water-borne epidemic type; the blood or needle associated type; and the sporadically occurring (community acquired) type. However, the number of agents which may cause NANBH is unknown.

Clinical diagnosis and identification of NANBH has been accomplished primarily by exclusion of other viral markers. Among the methods used to detect putative NANBH antigens and antibodies are agar-gel diffusion, counter-immunoelectrophoresis, immunofluorescence microscopy, immune electron microscopy, radioimmunoassay, and enzyme-linked immunosorbent assay. However, none of these assays has proved to be sufficiently sensitive, specific, and reproducible to be used as a diagnostic test for NANBH.

For the development of a specific and sensitive method to enable a reliable diagnosis to be made in various phases of the infection with NANBH it is of great importance to identify immuno-dominant viral epitopes of this type.

European Patent Application EP-A-0388232 discloses the complete amino acid sequence of the hepatitis C virus open reading frame.

In our co-pending application EPA 90.200.775.6 we disclosed a peptide with 15 amino acids and an amino acid sequence which is immunochemically reactive with NANBH antibodies. Furthermore very potent fragments of said pentadecapeptide especially the nonapeptide with amino acid sequence Arg-Lys-Thr-Lys-Arg-Ser-Thr-Asn-Arg were disclosed.

The present invention is dealing with new peptides having improved immunochemical reactivity with NANBH-antibodies.

The new peptides according to the invention are characterised by the following amino acid sequence: 
X - a- Lys - b - c - Asn - d - e - f - g - Y          I
    5    6     7    8     9     10   11   12   13
in which
- "a": represents the amino acid residues: Arg, Gln, Glu, or pyroGlu
- "b": represents Thr or Trp,
- "c": represents Lys, Val, Glu, Thr or Ser,
- "d": represents Ser, Asn or Ile,
- "e": represents Thr or Arg,
- "f": represents Asn or Met,
- "g": represents Arg, Lys, Tyr, Ile or Met and wherein
- X: represents hydrogen or an amino acid or a di-, tri-or tetra-peptide indicated by the symbols:
h₄-, h₃-h₄-, h₂-h₃-h₄- or h₁-h₂-h₃-h₄-
in which
- h₄: is the amino acid residue Gln or Val, h₃ is the amino acid residue Gln, Glu, His, Met or Pro,
- h₂: is the amino acid residue Thr or Ser and
- h₁: is the amino acid residue Arg, Gly, Lys or Trp
- Y: represents hydroxy or an amino acid or dipeptide indicated by the symbols: -h₁₄ or -h₁₄-h₁₅ in which
- h₁: ₄ is an amino acid residue selected from Arg and Glu, and
- h₁₅: is selected from Arg, Glu and Lys.

Above peptides have improved immunochemical reactivity with NANBH antibodies when compared to the pentadeca peptide with the sequence Arg-Thr-Gln-Gln-Arg-Lys-Thr-Lys-Arg-Ser-Thr-Asn-Arg-Arg-Arg as disclosed in our co-pending application EPA 90.200.775.6.

By improved immunochemical reactivity is meant that peptides exhibit an improved immunochemical reactivity in comparison with the referenced pentadeca-peptide if the use of the new peptides in a testprocedure as described in example 4 or in any other suitable immunoassay results in a higher specificity and/or sensitivity of the test.

Preferred peptides according to the invention showing excellent affinity towards NANBH antibodies, are peptides according to the above general formula, in which either alone or in combination
X represents hydrogen or the tetrapeptide h₁-h₂-h₃-h₄, and
Y represents hydroxy or the dipeptide -h₁₄-h₁₅, whereby h₁₅ is preferably selected from Arg and Lys.

The invention further comprises fragments of the peptides of formula I which are still immunochemically reactive with NANBH antibodies and (poly)peptides which contain the said peptides of formula I or active fragments thereof as an essential part of the amino acid sequence, provided that the active fragments of the peptide of formula I have the amino acid residue Asn at a position which corresponds to position 9 of the peptides of formula I.

In addition to the above peptides, functional derivatives of these peptides are also considered to belong to the peptides according to the instant invention. Functional derivatives of the peptides are meant to include
a) acid addition salts of the peptides;
b) amides of the peptides and specifically the C-terminal amides;
c) esters and specifically C-terminal esters and
d) N-acyl derivatives, specifically N-terminal acyl derivatives and in particular N-acetyl derivatives.

The peptides according to the invention are particularly suitable for use in a diagnostic method for the determination of the presence of NANBH antigens or NANBH antibodies in a test fluid.

In contrast to the natural NANBH, the peptides according to the invention have the advantage that these are of a safe non-infectious origin. Moreover, the present peptides have a particular high affinity to NANBH antibodies, which renders the present peptides extremely suitable for use in the above said diagnostic test methods.

A further embodiment of the present invention is therefore an immunochemical reagent (to be explained later on), which reagent contains one or more of the peptides of the invention.

The invention further comprises a method for the detection of antibodies directed against NANBH in a test fluid, whereby one or more of the peptides according to the invention are used.

The invention also relates to a method for the detection of NANBH in a test fluid, using one or more of the peptides according to the invention.

Finally, the invention relates to a test kit to be used for carrying out an immuno-assay, this test kit containing at least one immunochemical reagent according to the invention.

It is within the scope of this invention to use the new nucleotide sequence or part(s) thereof coding for the amino acid sequence(s) according to the invention, so-called primers, as basis of a test to detect NANBH DNA or RNA by a nucleic acid amplification technique for instance the polymerase chain reaction (PCR) or the nucleic acid sequence based amplification (NASBA), as described in USP 4,683,202 and EP 329,822, respectively.

A part of the invention includes a test amplification kit for carrying out an amplification and detection method described above.

Moreover, a peptide or fragment thereof according to the invention can be used in suitable pharmaceutical dosage forms in the prevention and/or treatment of NANB Hepatitis-disease. The preparation of vaccines thus obtained using such a peptide or fragment thereof as active ingredients, can be accomplished by one skilled in the art.

The preparation of the peptides according to the invention is effected by means of one of the known organic chemical methods for peptide synthesis or with the aid of recombinant DNA techniques. This latter method involves the preparation of the desired peptide by means of bringing to expression a recombinant polynucleotide with a polynucleotide sequence which is coding for one or more of the peptides in question in a suitable micro-organism as host.

The organic chemical methods for peptide synthesis are considered to include the coupling of the required amino acids by means of a condensation reaction, either in homogeneous phase or with the aid of a so-called solid phase.

The condensation reaction can be carried out as follows:
a) condensation of a compound (amino acid, peptide) with a free carboxyl group and protected other reactive groups with a compound (amino acid, peptide) with a free amino group and protected other reactive groups where one of the protecting groups also may be a (derivatised) solid support, in the present of a condensation agent,
b) condensation of a compound (amino acid, peptide) with an activated carboxyl group and free or protected other reaction groups with a compound (amino acid, peptide) with a free amino group and free or protected other reactive groups, where one of the protecting groups also may be a (derivatised) solid support.

Activation of the carboxyl group can take place, inter alia, by converting the carboxyl group to an acid halide, azide, anhydride, imidazolide or an activated ester such as the
N-hydroxy-succinimide, N-hydroxybenzotriazole, p-nitrophenyl,
3,4-dihydro-3-hydroxy-4-oxo-1,2,3-benzotriazine (ODhbt) or pentafluorophenyl (OPfp) ester.

The most common methods for the above condensation reactions are: the carbodiimide method, the BOP method [benzotriazolyloxytris (dimethylamino) phosphonium hexafluoro phosphate] the azide method, the mixed anhydride method and the method using activated esters, such as described in The Peptides, Analysis, Synthesis, Biology Vol. 1-3 (Ed. Gross, E. and Meienhofer, J.) 1979, 1980, 1981 (Academic Press, Inc.).

The reactive groups which may not participate in the condensation reaction are, as stated, effectively protected by groups which can be removed again very easily by hydrolysis with the aid of acid, base or reduction. Thus, a carboxyl group can be effectively protected by, for example, esterification with methanol, ethanol, teriary butanol, benzyl alcohol or p-nitrobenzyl alcohol and amidation with amines or derivatives of alcohols and amines linked to solid supports.

Groups which can effectively protect an amino group are the ethoxycarbonyl, benzyloxycarbonyl, t-butoxy-carbonyl, 9-fluorenyl-methoxycarbonyl(Fmoc) or p-methoxy-benzyloxycarbonyl group, or an acid group derived from a sulphonic acid, such as the p-toluene-sulphonyl, penta-methylbenzene sulfonyl (Pms), 4-methoxy-2,3,6-trimethyl-benzene sulfonyl (Mtr) or 1,2,5,7,8-pentamethylchroman-6-sulfonyl (Pmc) group, but other groups can also be used, such as substituted or unsubstituted aryl or aralkyl groups, for example benzyl and triphenylmethyl, or groups such as ortho-nitrophenyl-sulphenyl and 2-benzoyl-1-methylvinyl.

A more extensive account of possible protecting groups can be found in The peptides, Analysis, Synthesis, Biology Vol. 1-9 (Eds. Gross, Udenfriend and Meienhofer) 1979-1987 (Academic Press, Inc.).

Preparation of the abovementioned peptides according to the invention using the "solid phase" method, is for example described in J.Am.Chem.Soc., 85, 2149 (1963) and Int.J.Peptide Protein Res., 35, 161-214 (1990). The coupling of the amino acids of the peptide to be prepared usually starts from the carboxyl end side. For this method a solid phase is needed on which there are reactive groups or on which such groups can be introduced. This can be, for example, a copolymer of benzene and divinylbenzene with reactive chloromethyl groups, or a polymeric solid phase rendered reactive with hydroxymethyl or amine-functions.

A particularly suitable solid phase is, for example, the p-alkoxybenzyl alcohol resin (4-hydroxymethyl-phenoxy-methyl-copolystrene-1% divinylbenzene resin), described by Wang (1974) J.Am.Chem.Soc., 95, 1328. After synthesis the peptides can be split from this solid phase under mild conditions. Other suitable supports are derivatised poly-ethylene or polypropylene rods as described by Geysen, Proc. Natl. Acad. Sci., 81, 3998 (1984) and Proc. Natl. Acad. Sci., 82, 178 (1985).

After synthesis of the desired amino acid sequence, either in solution or on a solid support, the protective groups can be split off by various conventional methods, depending on the nature of the particular group, for example with the aid of trifluoro-acetic acid or by mild reduction, for example with hydrogen and a catalyst, such as palladium, treatment with a base as for example piperidine or hydroxide ions, or with HBr in glacial acetic acid.

If the peptide is synthesized on a solid support from which it can be detached this can be achieved, depending on the type of linker, for example, with trifluoro-acetic acid, trifluoromethanesulphonic acid or with methanesulphonic acid dissolved in trifluoroacetic acid, transesterification with a lower alcohol, preferably methanol or ethanol, in which case a lower alkyl ester of the peptide is formed directly. Likewise, splitting with the aid of ammonia gives the amide of a peptide according to the invention.

As already indicated above, the peptide according to the invention can likewise be prepared with the aid of recombinant DNA techniques. This possibility is of importance particularly when the peptide is incorporated in a repeating sequence ("in tandem") or when the peptide can be prepared as an essential constituent of a (much larger) protein or polypeptide. This type of preparation of the peptide therefore likewise falls within the scope of the invention. For this purpose, as a constituent of a recombinant DNA, a polynucleotide is used which codes for the peptide according to the invention and which, furthermore, is substantially free from polynucleotide segments, which in the naturally occurring NANBH genome flank the polynucleotide sequence indicated above.

A polynucleotide of this type, which is coding for the peptide according to the invention, and a recombinant DNA in which this polynucleotide is incorporated likewise fall within the scope of the invention.

The peptides or fragments thereof prepared and described above are used to produce antibodies, both polyclonal and monoclonal. Monoclonal antibodies directed against peptides according to the invention can be readily produced by one skilled in the art.

Making monoclonals by hybridomas is well known. Cell fusion, immortal antibody-producing cell lines can be created while also other techniques are available such as direct transformation of B-lymphocytes with oncogenic DNA or transfection with Epstein-Barr Virus.

Antibodies, both monoclonal and polyclonal, directed against peptides according to the invention are very suitable in diagnosis, while those antibodies which are neutralizing are very useful in passive immunotherapy. Especially monoclonal antibodies may be used to raise anti-idiotype antibodies. Techniques for raising anti-idiotype antibodies are known in the art.

Said anti-idiotype antibodies are also useful for prevention and/or treatment of Non-A, Non-B Hepatitis, as well as for the elucidation of important epitopic regions of NANBH-antigens.

The "immunochemical reagent" according to the invention usually consists of one or more peptides according to the invention and a suitable support or a labelling substance.

A support that may be used in this respect is, for example, a carrier protein such as BSA, the inner wall of a microtest well or a cuvette, a tube or capillary, a membrane, filter, test strip or a particle such as, for example, a latex particle, an erythrocyte, a dye sol, a metal sol or metal compound as sol particle.

A labelling substance that may be used is i.a. a radioactive isotope, a fluorescent compound, a labelling protein such as (an enzyme), an enzyme, a dye sol, metal sol or metal compound as sol particle.

The said immunochemical reagent is the essential component in a method for the detection of antibodies directed against NANBH in a test fluid. The immunochemical reagent is therefore brought into contact with the said test fluid whereby an immunochemical reaction takes place resulting in the formulation of an immune complex between the peptide (according to the invention) and the NANBH antibodies. Depending on the nature and further characteristics of the immunochemical reagent the immunochemical reaction that takes place is a socalled sandwich reaction, an agglutination reaction, a competition reaction or an inhibition reaction.

The immune complex formed as a result of the above reaction is a (direct or indirect) measure for the presence of antibodies in the test fluid.

The immunochemical reagent according to the invention can also be used for the detection of NANBH antigen in a test fluid. In this detection method the test fluid is contacted with anti-NANBH and subsequently or simultaneously with the immunochemical reagent according to the invention.

A particularly suitable method for the detection of NANBH in a test fluid is based on a competition reaction between a peptide according to the invention provided with a labelling substance and a NANBH antigen (present in the test fluid), whereby the peptide and the antigen are competing with the antibody directed against NANBH attached to a solid support.

A test kit according to the invention comprises, as an essential constituent, an immunochemical reagent as described above. In carrying out a sandwich reaction for the detection of NANBH antibodies the test kit may comprise, for example,
1) the peptide according to the invention attached to a solid support, (for example the inner wall of a microtest well) and
2) either a labelled peptide according to the invention or a labelled anti-antibody.

In carrying out a competition reaction for detection NANBH antibodies, the test kit may comprise
1) the peptide according to the invention attached to a solid support, and
2) a labelled antibody directed against NANBH preferably a monoclonal antibody directed against said peptide.

In carrying out an agglutination reaction the test kit comprises an immunochemical reagent which consists of a peptide according to the invention attached to particles or sols.

A test kit for the detection of NANBH antigen comprises, for example, a labelled peptide according to the invention and an antibody directed against NANBH, which is attached to a solid support.

### Example I

H-Arg-Thr-Gln-Gln-Arg-Lys-Thr-Lys-Asn-Ser-Thr-Asn-Arg-
1 5 10
Arg-Arg-OH
15

The pentadecapeptide with the sequence as shown in the heading was prepared by stepwise solid phase peptide synthesis. The synthesis was carried out using a VEGA Coupler 250 C automated peptide synthesizer or a Labortec SP640 semi-automatic peptide synthesizer, employing a p-benzyloxybenzyl alcohol resin (Wang-resin; 0.6-0.7 mmoles/g, Bachem AG, Switzerland) and N^{α}-Fmoc-protected (Fmoc, 9-fluorenyl-methyloxy-carbonyl) amino acids.

The synthesis started by the coupling of Fmoc-Arg(Pmc)-OH to the resin using DCC (dicyclohexylcarbodiimide, 1 equivalent), HOBt (1-hydroxybenzotriazole, 2 equivalents) and DMAP (N,N-dimethylaminopyridine, 1 equivalent) in DMF-dichloromethane (1:1, vol/vol) at 4 ^{o}C for 18 hours. Unreacted alcohol functions on the resin were then blocked by benzoylation using benzoylchloride-pyridine for 2 hours.

The resulting Fmoc-Arg(Pmc)-resin (0.38 mmol/g) was successively treated three times with 20% piperidine in DMF for 6 min, in order to remove the Fmoc-group. The protected pentadecapeptide was then prepared on the H-Arg(Pmc)-resin by successive coupling steps of the Fmoc-amino acids, as dictated by the amino acid sequence. The following side chain protecting groups were used: Pmc (2,2,5,7,8-pentamethylchroman-6-sulfonyl-) for Arg; tBu (tert.butyl) for Thr and Ser; Boc(t-butyloxycarbonyl) for Lys and Trt (trityl) for Asn and Gln.

Each coupling step was performed using 3 equivalents each of Fmoc-amino acid, BOP (= benzotriazolyloxy-tris(dimethylamino)-phosphonium hexafluorophosphate), HOBt and 4,5 equivalents of DIPEA (= N,N-diisopropyl-ethylamine) in 12-15 ml of DMF per gram resin for 15 min, followed by 3 cycles of washings (one min each) with DMF and ethanol. Completeness of the coupling reaction was monitored by the ninhydrin test of Kaiser (Anal. Biochem. 34, 595-598, 1970). A positive ninhydrin reaction was observed following coupling of Ser¹⁰ and Arg⁵. In each case the coupling reaction was repeated using one equivalent of corresponding Fmoc-amino acid, BOP, HOBt and 1,5 equivalent of DIPEA for 30 min. Any remaining free amino groups were then blocked by acetylation using acetic anhydride-DMF (5:95; vol/vol) for 10 min and subsequent washings with DMF and ethanol (1 min each), respectively.

After each synthesis cycle the N^{α}-Fmoc-protecting group was removed by treatment with 25% piperidine in DMF as described above.

After completion of the synthesis, the resulting fully protected pentadecapeptide resin was treated in a mixture of trifluoro acetic acid - water - phenol - thioanisole - ethanedithiol (82:5:5:5:2,5 vol/vol) for 18 hours at room temperature, in order to effect release of the peptide from the resin with simultaneous removal of all protecting groups. The crude peptide was isolated following precipitation upon addition of the reaction mixture to diethyl ether. The pentadecapeptide was purified by HPLC on C₁₈-silica using a gradient of acetonitrile in 0.1 M phosphate buffer at pH 2.1.

### Example II

In a corresponding manner were prepared:

H-Lys-Thr-Gln-Gln-Arg-Lys-Thr-Lys-Asn-Ser-Thr-
-Asn-Arg-Arg-Arg-OH
H-Arg-Thr-Gln-Gln-Arg-Lys-Trp-Lys-Asn-Ser-Thr-
-Asn-Arg-Arg-Arg-OH
H-Gly-Thr-Gln-Gln-Arg-Lys-Trp-Lys-Asn-Ser-Thr-
-Asn-Arg-Arg-Arg-OH.

### Example III

H-Arg-Lys-Thr-Lys-Asn-Ser-Thr-Asn-Arg-OH and derivatives thereof.

The above nonapeptide was prepared by stepwise solid phase peptide synthesis, essentially following the process described in example I. Adaptations were made in order to allow the simultaneous synthesis of large numbers of sequences e.g. derivatised polymer rods (loading 150-300 nmol amine/pin) mounted in a block, both constructed of inert material as polyethylene or polypropylene were used as the solid support for the synthesis of the peptides instead of beaded polystyrene based resins. All washing, coupling and deprotection steps were performed by immersing the rods (mounted in the block) in containers with the appropriate solutions.

Preactivated N^{α}-Fmoc-amino acids (OPfp or ODhbt esters) in the presence of HOBt, both in a concentration of 30 mmoles/litre were used for chain elongation. The N^{α}-Fmoc-groups were removed after each synthesis cycle with 20% piperidine in DMF. The following side chain protecting groups were used: -Pmc for Arg; -tBu for Ser, Thr and Glu; Boc for Lys; Trt for Asn and Gln.

A typical synthesis cycle consisted of the following steps:

| | | |
|---|---|---|
| 1. | Deprotection (20% piperidine in DMF) | 30 min |
| 2. | Wash: | |
| | DMF | 1 x 5 min |
| | Methanol | 4 x 2 min |
| 3. | Air dry | 10 min |
| 4. | Wash: DMF | 1 x 5 min |
| 5. | Coupling (30 mM Fmoc amino acid active ester and 30 mM HOBtin DMF) | 18 hours |
| 6. | Wash: | |
| | DMF | 1 x 2 min |
| | Methanol | 4 x 2 min |
| | DMF | 1 x 2 min |
| 7. | Air dry | 1 x 2 min |

After completion of the synthesis the N^{α}-Fmoc-group was removed by treatment with 20% piperidine in DMF as described above and the other protective groups were removed by treatment with trifluoro acetic acid-phenol-ethanedithiol = 95 - 2,5 - 2,5 (v/m/v) for 4 hours at room temperature, which leaves the bond between peptide and solid support intact. The pins were subsequently washed with methylene chloride, 5% diisopropylamine in methylenechloride, methylene chloride and methanol and dried.

The following peptides were prepared:
1. H-Arg-Lys-Thr-Lys-Asn-Ser-Thr-Asn-Arg-OH
2. H-Glu-Lys-Thr-Lys-Asn-Ser-Thr-Asn-Arg-OH
3. H-pyroGlu-Lys-Thr-Lys-Asn-Ser-Thr-Asn-Arg-OH
4. H-Arg-Lys-Trp-Lys-Asn-Ser-Thr-Asn-Arg-OH
5. H-Arg-Lys-Thr-Thr-Asn-Ser-Thr-Asn-Arg-OH
6. H-Arg-Lys-Thr-Glu-Asn-Ser-Thr-Asn-Arg-OH
7. H-Arg-Lys-Thr-Lys-Asn-Asn-Thr-Asn-Arg-OH
8. H-Arg-Lys-Thr-Lys-Asn-Ile-Thr-Asn-Arg-OH
9. H-Arg-Lys-Thr-Lys-Asn-Ser-Arg-Asn-Arg-OH
10. H-Arg-Lys-Thr-Lys-Asn-Ser-Thr-Met-Arg-OH
11. H-Arg-Lys-Thr-Lys-Asn-Ser-Thr-Asn-Lys-OH
12. H-Gln-Arg-Lys-Thr-Lys-Asn-Ser-Thr-Asn-Arg-OH
13. H-Gln-Arg-Lys-Thr-Lys-Asn-Ser-Thr-Asn-Arg-Arg-OH.

### Example IV

The pentadecapeptide of example I was covalently coupled to bovine serum albumine (BSA) by a standard glutaric acid conjugation method. Equal volumes at the following solutions in 100 mM phosphate buffer pH 7.0 were added together: 0,3 mM BSA, 4 mM pentadecapeptide and 7 mM glutaric acid.

After overnight incubation at room temperature the reaction was stopped by the addition of 0,1 volume of 40% sucrose, 1 M glycine, 0,08 M NaH₂PO₄, 0,025 M NaBH₄, pH 7.0 and incubation for 1 hour at room temperature. The peptide-BSA conjugate was diluted to 20 »g/ml in 0,05 M Carbonate buffer pH 9.6 and 135 »l of this solution was given into a well of a microtiter plate. Adsorbtion of the peptide-BSA conjugate was allowed to proceed overnight at 4 °C. The plates were then emptied and residual binding sites were blocked with a solution of 0,1 M Tris pH 7.4, 0,2% BSA and 0,03 M KI for 20 min. at room temperature. Plates were then air-dried overnight at room temperature and stored on silicagel at 4 °C. For the detection of antibodies specific for NANBH, the serum sample was diluted 1:10 in sample diluent (PBS/20% normal goat serum/1% Triton X100) pipetted into the well (100 »l per well) and incubated for 1 hour at 37 °C.

After washing the wells with PBS/0.05% Tween 20^{(R)} the bound human antibodies were detected with goat anti-human immunoglobulin labeled with peroxidase (100 »l per well, 1 h at 37 °C) diluted in sample diluent. The plates were washed 4 times with PBS/0.05% Tween 20^{(R)}. TMB was added (100 »l per well) as a substrate for the peroxidase enzyme and the reaction was allowed to proceed for 30 min. at room temperature. The reaction was stopped by adding 100 »l 2M H₂SO₄ to each well. The yellow color was read at 450 nm in an Organon Teknika microelisa reader.

### Example V

The peptides of example III were also tested for reactivity with sera from patients with NANBH.
The nonapeptides 1 to 13 were coupled to a solid support essentially in the same manner as described in example IV. Immune reactivity with serum samples obtained from patients with NANBH was established as described in example IV.

## Claims (Claims for the following Contracting State(s): AT, BE, CH, DE, DK, FR, GB, IT, LI, NL, SE)

1. A polypeptide comprising the sequence **Arg-Lys-Thr-Lys-Asn-Ser-Thr-Asn-Arg** as well as acid addition salts, amides, esters and N-acyl derivatives thereof, which polypeptide is immuno-reactive with NANBH-antibodies.

2. Immunochemical reagent comprising the peptide of claim 1.

3. Method for the detection of NANBH-antibodies in a test fluid, comprising contacting the immunochemical reagent of claim 2 with the test fluid, and detecting the presence of immune complexes formed between the immunochemical reagent and the antibodies in the test fluid.

4. Method for the detection of NANBH in a test fluid comprising contacting the immunochemical reagent of claim 2 with the test fluid and NANBH-antibodies, and detecting the presence of immune complexes formed between the immunochemical reagent and NANBH-antibodies.

5. Test kit for the detection of NANBH or NANBH-antibodies comprising the immunochemical reagent of claim 2.

## Claims (Claims for the following Contracting State(s): ES, GR)

1. Method for the production of a polypeptide comprising the sequence **Arg-Lys-Thr-Lys-Asn-Ser-Thr-Asn-Arg** as well as acid addition salts, amides, esters and N-acyl derivatives thereof, which polypeptide is immunoreactive with NANBH-antibodies, using one of the known organic chemical methods for peptide synthesis or using recombinant DNA techniques.

2. Method for the detection of NANBH-antibodies in a test fluid, characterized in that an immunochemical reagent comprising a polypeptide according to claim 1 is brought into contact with the test fluid, and the presence of any immune complexes formed between the immunochemical reagent and the antibodies in the test fluid is detected.

3. Method for the detection of NANBH in a test fluid, characterized in that an immunochemical reagent comprising a polypeptide according to claim 1 is brought into contact with the test fluid and NANBH-antibodies, after which the presence of immune complexes formed is detected and the presence of HCV in the test fluid is determined.

## Patentansprüche (Patentansprüche für folgende(n) Vertragsstaat(en): AT, BE, CH, DE, DK, FR, GB, IT, LI, NL, SE)

1. Ein Polypeptid, das die Sequenz **Arg-Lys-Thr-Lys-Asn-Ser-Thr-Asn-Arg** als auch Säureadditionssalze, Amide, Ester und N-Acylderivate davon umfasst, wobei das Polypeptid mit NANBH-Antikörpern immunologisch reaktionsfähig ist.

2. Immunochemisches Reagenz, das das Peptid von Anspruch 1 umfasst.

3. Verfahren zum Nachweis von NANBH-Antikörpern in einer Testflüssigkeit, das ein in Kontaktbringen des immunochemischen Reagenz gemäss Anspruch 2 mit der Testflüssigkeit und Nachweisen der Anwesenheit von Immunkomplexen, die sich zwischen dem immunochemischen Reagenz und den Antikörpern in der Testflüssigkeit gebildet haben, umfasst.

4. Verfahren zum Nachweis von NANBH in einer Testflüssigkeit, das ein in Kontaktbringen des immunochemischen Reagenz gemäss Anspruch 2 mit der Testflüssigkeit und NANBH-Antikörpern und Nachweisen der Anwesenheit von Immunkomplexen, die sich zwischen dem immunochemischen Reagenz und NANBH-Antikörpern gebildet haben, umfasst.

5. Testgarnitur zum Nachweis von NANBH oder NANBH-Antikörpern, die das immunochemische Reagenz von Anspruch 2 umfasst.

## Patentansprüche (Patentansprüche für folgende(n) Vertragsstaat(en): ES, GR)

1. Verfahren zur Herstellung eines Polypeptids, das die Sequenz **Arg-Lys-Thr-Lys-Asn-Ser-Thr-Asn-Arg** als auch Säureadditionssalze, Amide, Ester und N-Acylderivate davon umfasst, wobei das Polypeptid mit NANBH-Antikörpern immunologisch reaktionsfähig ist, unter Verwendung eines der bekannten organisch-chemischen Verfahren zur Peptidsynthese oder unter Verwendung rekombinanter DNA-Verfahren.

2. Verfahren zum Nachweis von NANBH-Antikörpern in einer Testflüssigkeit, dadurch gekennzeichnet, dass ein immunochemisches Reagenz, das ein Polypeptid gemäss Anspruch 1 umfasst, mit der Testflüssigkeit in Kontakt gebracht wird und die Anwesenheit aller Immunkomplexe, die sich zwischen dem immunochemischen Reagenz und den Antikörpern in der Testflüssigkeit gebildet haben, nachgewiesen wird.

3. Verfahren zum Nachweis von NANBH in einer Testflüssigkeit, dadurch gekennzeichnet, dass ein immunochemisches Reagenz, das ein Polypeptid gemäss Anspruch 1 umfasst, mit der Testflüssigkeit und NANBH-Antikörpern in Kontakt gebracht wird, wonach die Anwesenheit von gebildeten Immunkomplexen nachgewiesen und die Anwesenheit von HCV in der Testflüssigkeit bestimmt wird.

## Revendications (Revendications pour l'(les) Etat(s) contractant(s) suivant(s): AT, BE, CH, DE, DK, FR, GB, IT, LI, NL, SE)

1. Un polypeptide comprenant la séquence Arg-Lys-Thr-Lys-Asn-Ser-Thr-Asn-Arg ainsi que ses sels d'addition d'acide, ses dérivés amides, esters et N-acyles, ce polypeptide étant immunoréactif avec des anticorps NANBH.

2. Réactif immunochimique comprenant le peptide selon la revendication 1.

3. Méthode de détection d'anticorps NANBH dans un fluide à tester comprenant la mise du réactif immunochimique de la revendication 2 au contact du fluide à tester et à la détection de la présence de complexes immuns formés entre le réactif immunochimique et les anticorps dans le fluide à tester.

4. Méthode de détection de NANBH dans un fluide à tester comprenant la mise du réactif immunochimique de la revendication 2 au contact du fluide à tester et d'anticorps NANBH et la détection de la présence de complexes immunes formés entre le réactif immunochimique et les anticorps NANBH.

5. Kit de test pour la détection de NANBH ou d'anticorps NANBH comprenant le réactif immunochimique selon la revendication 2.

## Revendications (Revendications pour l'(les) Etat(s) contractant(s) suivant(s): ES, GR)

1. Procédé de préparation d'un polypeptide comprenant la séquence Arg-Lys-Thr-Lys-Asn-Ser-Thr-Asn-Arg ainsi que ses sels d'addition d'acide, ses dérivés amides, esters et N-acyles, ce polypeptide étant immunoréactif avec les anticorps NANBH, ce procédé utilisant l'une des méthodes de chimie organique connues pour la synthèse des peptides ou utilisant des techniques d'ADN recombinant.

2. Procédé pour la détection d'anticorps NANBH dans un fluide à tester, caractérisé en ce que l'on met au contact du fluide à tester un réactif immunochimique comprenant un polypeptide selon la revendication 1 et en ce que l'on détecte la présence de tout complexe immun formé entre le réactif immunochimique et les anticorps dans le fluide à tester.

3. Méthode de détection de NANBH dans un fluide à tester, caractérisé en ce que un réactif immunochimique comprenant un polypeptide selon la revendication 1 est mis au contact du fluide à tester et des anticorps NANBH, ce après quoi on détecte la présence de complexes immuns et on détermine la présence de HCV dans le fluide à tester.
